# EUROPÄISCHE PATENTANMELDUNG

(11) **EP 0 807 435 A2**
(43) Veröffentlichungstag der Anmeldung: **19.11.1997**
(21) Anmeldenummer: 97108124.5
(22) Anmeldetag: 20.05.1997
(51) Int. Cl.: A61K 31/70, A61K 31/35

(54) **Indirekter HMG-CoA-Reduktase-Hemmer**

(30) Priorität: 17.05.1996 DE 19620001
(71) Anmelder: Sertürner Arzneimittel GmbH, 33332 Gütersloh (DE)
(72) Erfinder: Gebhardt, R.,Prof.-Dr. Eberhard-Karls-Uni.Tübingen, 72076 Tübingen (DE)
(74) Vertreter: VOSSIUS & PARTNER

(57) **Zusammenfassung**

Die Erfindung betrifft die Verwendung von Luteolin, seiner Derivate und verwandter Verbindungen zur Herstellung eines Arzneimittels zur indirekten Hemmung der HMG-CoA-Reduktase.

## Beschreibung

Die Erfindung betrifft die Verwendung von Luteolin, seiner Derivate und verwandter Verbindungen zur Herstellung eines indirekten Inhibitors der Hydroxymethylglutaryl-CoA-Reduktase (HMG-CoA-Reduktase).

Herz-Kreislauferkrankungen sind die häufigste Krankheits- und Todesursache in der Bundesrepublik Deutschland. Hyperlipoproteinämien als Hauptrisikofaktor für Arteriosklerose, Herzinfarkt und andere kardiovaskuläre Krankheiten stellen die präventive und kurative Medizin vor zunehmend größere Probleme, da die Möglichkeiten der medikamentösen Intervention auf Grund der Risiken der synthetischen Lipidsenker begrenzt sind. Präventive Anwendungen von synthetischen Lipidsenkern scheiden auf Grund des Nebenwirkungsrisikos bislang aus.

So werden mit den verfügbaren synthetischen direkten HMGCoA-Reduktase-Hemmern wie Mevastatin, Simvastatin, Pravastatin, Lovastatin eine Beseitigung des Lipoproteinrisikos nur unter dem Risiko von teils schwerwiegenden Nebenwirkungen erreicht. Das gilt auch für Clofibrinsäure sowie deren Derivate und Analoga, Probucol, Cholestyramin, Colestipol und Nikotinsäure sowie Nicotinylalkohol. Die Klinik der unerwünschten Arzneimittelwirkungen synthetischer Lipidsenker sind hinreichend bekannt (1, 2, 3, 4), als problematisch wird angesehen, daß die Langzeitsicherheit von synthetischen Cholesterinsynthesehemmern bislang nicht geklärt ist (4, 1). Dabei ist die Zielgruppe für die Anwendung von Lipidsenkern groß. Nach aktuellen Schätzungen sind z.B. 1/4 der männlichen Bevölkerung zwischen 45 und 64 Jahren Kandidaten für eine Lipidsenker-Behandlung.

Bei Frauen nach der Menopause kann eine Östrogensubstitutionstherapie eine Senkung des LDL-Cholesterins bewirken, allerdings steigt das Risiko für Mammakarzinome an.

Die Anwendung von synthetischen Lipidsenkern ist darüber hinaus derzeit weltweit mit hohen Behandlungskosten verbunden.

Obwohl durch Senkung erhöhter Cholesterinwerte Herzinfarktrate und Gesamtsterblichkeit reduziert werden (5), erhalten lediglich ein kleiner Bruchteil der Patienten mit erhöhten Cholesterinwerten eine Behandlung, von diesen erreichten wiederum nur weniger als die Hälfte die von der Europäischen Artherosklerose-Gesellschaft gesteckten Zielwerte (6). Bei Cholesterinkonzentrationen ≥ 240 mg/dl steigt die Koronarmortalität auf das Dreifache (7). Zahlreiche Interventionsstudien belegen grundsätzlich den Nutzen lipidsenkender Maßnahmen zur Absenkung des Koronarrisikos [Übersicht bei (8)]. Länder mit aktiven Maßnahmen zur Senkung des Koronarrisikos weisen einen Rückgang koronarer Todesfälle um 50 % auf, während Länder ohne entsprechende Maßnahmen eine Zunahme von 40 % verzeichnen (6).

Als Grenzwerte für die Konzentration des Gesamtcholesterin im Blut werden im allgemeinen 200 mg/dl angesehen (9). Die Interventionsgrenze wird wesentlich mitbestimmt durch das Vorhandensein weiterer Risikofaktoren, jedoch ist im allgemeinen erst ab Werten von ca. 250 mg/dl eine medikamentöse Intervention indiziert (10).

Aufgrund der Nebenwirkungen der herkömmlichen Lipidsenker, insbesondere auch wegen der Risiken bei Langzeittherapie, werden zur Normalisierung der Blutfette primär diätetische Maßnahmen empfohlen. Unterhalb einer Gesamtcholesterin-Blutkonzentration von 250 mg/dl kommen daher überwiegend zunächst lipidsenkende diätetische Maßnahmen zur Anwendung (10). Hierfür ist jedoch eine konsequente und disziplinierte Einhaltung der lipidsenkenden Diät über einen sehr langen Zeitraum erforderlich, so daß in sehr vielen Fällen derartige diätetische Maßnahmen scheitern. Der Erfolg diätetischer Maßnahmen ist nach heutiger Erkenntnis gering, weshalb nach diätunterstützenden medikamentösen Maßnahmen gesucht wird (12).

Dennoch werden im allgemeinen lediglich bei stärker erhöhten Cholesterinwerten von über ca. 250 mg/dl die Nebenwirkungen einer medikamentösen Therapie mit herkömmlichen Lipidsenkern in Kauf genommen. Nach neueren Erkenntnisen schließen auch deutlich niedrigere Gesamtcholesterinwerte ein Infarktrisiko keineswegs aus (3), so daß wirksame Maßnahmen auch bei leicht erhöhten Cholesterinwerten dringend geboten sind. Prospektivstudien haben gezeigt, daß das erhöhte Risiko für vorzeitige koronare Herzkrankheit nicht erst bei den ausgesprochenen Hyperlipidämien angesiedelt ist, sondern progressiv mit dem Cholesterinspiegel wächst. Die Senkung erhöhter Cholesterinwerte um 1 % führt nach heutiger Erkenntnis zu einem Rückgang des Koronarrisikos um 2 % (10).

In der Literatur wird angesichts der Erfolge der Sekundärprävention der koronaren Herzkrankheit durch lipidsenkende Maßnahmen zur Primärprävention aufgerufen (5). Primärprävention mit Medikamenten setzt jedoch voraus, daß die Wirkmechanismen nicht zu einer ungünstigen Beeinflussung des Fettstoffwechsels führen.

Obwohl erhöhte Cholesterinwerte einen bedeutsamen Risikofaktor für die Entstehung der Arteriosklerose beim Menschen darstellen, haben Cholesterin und das Enzym HMG-CoA-Reduktase, das Schlüsselenzym der Cholesterinsynthese, sehr wichtige physiologische Funktionen. So stellt Cholesterin beispielsweise die Vorstufen der Gallensäuren und der Steroidhormone dar und ist ein wesentlicher Bestandteil der Zellmembran und Myelinscheide. In sehr vielen Fällen ist somit eine komplette Hemmung dieses physiologisch wichtigen Enzyms auf Grund der damit verbundenen negativen Wirkungen nicht wünschenswert. Dies ist insbesondere bei leichteren Formen von Hyperlipidämien der Fall.

Da insbesondere die herkömmlichen direkt wirkenden HMG-CoA-Reduktase-Hemmer mit einem erhöhten Arzneimittelrisiko verbunden sind, ist eine strenge Indikationsstellung erforderlich. In vielen Fällen ist selbst bei einem erheblich erhöhten Cholesterinspiegel die Verwendung von HMG-CoA-Reduktase-Hemmern kontraindiziert. Des weiteren ist zu berücksichtigen, daß bei einzelnen Patienten die Verträglichkeit und Wirksamkeit der herkömmlichen Lipidsenker erheblich variieren, so daß selbst bei genauer Überwachung der Dosierung die Therapie mit den herkömmlichen Lipidsenkern, die ihre Wirkung durch eine direkte Hemmwirkung auf das Enzym HMG-CoA-Reduktase entfalten, mit einer Hypocholesterinämie (Hypolipoproteinämie), d.h. eine Absenkung des Cholesterins unter die Normgrenze verbunden sein kann. Die Hemmung der notwendigen intrazellulären Cholesterinsynthese kann nachteilige Folgen haben (11).

Aufgabe der vorliegenden Erfindung ist es somit, ein Arzneimittel bereitzustellen, das im Vergleich zu den herkömmlichen Lipidsenkern ebenso wirksam ist, jedoch aufgrund seines Wirkmechanismus mit weniger Nebenwirkungen behaftet ist und somit insbesondere auch zur Prophylaxe und Langzeittherapie von Dyslipoproteinämien, insbesondere Hyperlipoproteinämien wie Hypercholesterinämien verwendet werden kann, die normalerweise nicht mit den herkömmlichen HMG-CoA-Reduktase-Hemmern behandelt werden sollten.

Es wurde unerwarteterweise gefunden, daß das Flavonoid Luteolin (31,41,5-Tetrahydroxyflavon) und seine Derivate, insbesondere pharmakologisch verträgliche Verbindungen sehr wirksame indirekte Inhibitoren des Enzyms HMG-CoA-Reduktase darstellen.

Es konnte festgestellt werden, daß im Gegensatz zu den "klassischen" Lipidsenkern, die ihre Wirkung durch eine direkte Hemmwirkung auf das Enzym HMG-CoA-Reduktase entfalten, die "indirekten Inhibitoren", wie das erfindungsgemäße Luteolin selbst in sehr hohen Dosen das Enzym HMG-CoA-Reduktase nur teilweise, niemals aber zu 100 % hemmen. Diese Hemmwirkung wird über eine Modulation der physiologischen Regulationsmechanismen der HMG-CoA-Reduktase erreicht, wobei noch unklar ist, ob aktivierende Mechanismen (Dephosphorylierung etc.) gehemmt oder inaktivierende Mechanismen (Phosphorylierung, Enzymabbau, etc.) aktiviert werden.

Im Gegensatz zu den direkt wirkenden HMG-CoA-Reduktase-Hemmern haben das erfindungsgemäße Luteolin und seine Derivate auf Grund des indirekten Wirkmechanismus eine hohe therapeutische Breite.

Die lipidsenkende Wirkung von Luteolin und Luteolin-7-Glycosid im Tierversuch (Kaninchen) wird in Chem. Abstr. 76: 1080 BOj erwähnt, jedoch sind keinerlei Hinweise über das Wirkungsprofil bzw. des Wirkungsmechanismus dieser Substanzen zu entnehmen. Die festgestellte Senkung der Lipide betraf zudem keine natürlich entstandene Erkrankung, sondern experimentell durch Cholesterinkost (200 mg Cholesterin pro kg Körpergewicht über 60 Tage) erzeugte Erhöhungen der Blutfette bei Kaninchen.

Luteolin ist ein sekundärer Pflanzenmetabolit der in verschiedenen pflanzlichen Arzneidrogen, z.B. in Artischockenblättern in pharmazeutisch wirksamer Konzentration meist glykosidisch gebunden vorkommt. Die Isolierung von Luteolin aus pflanzlichen Extrakten ist in der Literatur ausführlich dokumentiert (vgl. z.B. Farmatsüa 21(39 (1972) 37), ebenso wie die de novo-Synthese von Luteolin und seiner Derivate (vgl. z.B. J.Chem.Soc. 1939, 91).

Obwohl verschiedenen Flavonoiden antioxidative, LDL-oxidationshemmende, lipidperoxidationshemmende und hepatoprotektive Eigenschaften zugeschrieben werden, gibt es keinerlei Hinweis, daß Luteolin und seine Derivate das erfindungsgemäße pharmakologische Wirkungsprofil aufweisen und somit zur Therapie von Stoffwechselerkrankungen verwendet werden können, die bislang einer medikamentösen Therapie aufgrund Nutzen/Risiko-Erwägungen nicht zugänglich waren.

Selbst ein chronisch leicht erhöhter Cholesterinspiegel wird als Risikofaktor von Herzkreislauferkrankungen angesehen. Erfindungsgemäß kann erstmals durch die indirekte Hemmwirkung auf die HMG-CoA-Reduktase eine wirksame medikamentöse Behandlung dieser Zustände als partieller oder vollständiger Diätersatz bereitgestellt werden.

Auch ist aufgrund der indirekten Hemmwirkung auf die HMG-CoA-Reduktase und der damit verbundenen geringeren Risiken eine Langzeittherapie sehr erfolgversprechend, da sie die Patientencompliance, d.h. die Einhaltung des Therapieplans durch den Patienten besser gewährleistet als strenge Diätregimes.

Darüber hinaus kann Luteolin erfindungsgemäß auch in geeigneter Dosierung zur Prophylaxe und Behandlung von Dyslipoproteinämien verwendet werden, bei denen die Anwendung von direkten HMG-CoA-Reduktase-Hemmern auf Grund des spezifischen Wirkmechanismus Risiken birgt oder kontraindiziert ist.

Luteolin kann erfindungsgemäß insbesondere zur Prophlaxe und Behandlung von Dyslipoproteinämien verwendet werden, bei denen eine Absenkung der Cholesterinkonzentrationen unter die Normgrenze (sog. Hypocholesterinämie, Hypolipoproteinämie) vermieden werden muß.

Luteolinhaltige Phytopharmaka, insbesondere Artischockenblätterextrakte, sind bekannt. Derartige Extrakte enthalten eine Vielzahl pharmakologisch wirksamer Bestandteile. Der Anteil an Luteolin und seiner Glykoside in diesen Extrakten liegt üblicherweise zwischen 0,5 bis 2,0 Gew. %. Sie werden für verschiedene Indikationen arzneilich verwendet, insbesondere zur Behandlung dyspeptischer Beschwerden. Auch ist eine lipidsenkende Wirkung derartiger Extrakte beschrieben worden, wobei der lipidsenkende Effekt (unzutreffenderweise) auf den Wirkstoff Cynarin zurückgeführt wird.

Erfindungsgemäß wurde überraschenderweise an Hand von Messungen des Einbaus von ¹⁴C-markiertem Acetat in die Fraktion der nicht-verseifbaren neutralen Lipide von kultivierten Rattenhepatocyten nachgewiesen, daß Luteolin, seine Derivate und verwandte Verbindungen konzentrationsabhängig die Neusynthese von Cholesterin in den Leberzellen vermindern. Bei Verwendung eines Luteolin-7-0-glucosid-haltigen Artischockenblätterspezialextrakts im Kulturmedium in einer Konzentration zwischen 0,02 und 0,1 mg/ml tritt eine durchschnittliche Hemmung (Plateau) von ca. 20% ein, ohne daß zytotoxische Wirkungen nachweisbar wären. Bei höheren Konzentrationen (bis 1 mg/ml) tritt eine noch stärkere Hemmung auf (maximal 85 %), ohne daß zytotoxische Wirkungen nachweisbar sind, die frühestens oberhalb von 2 mg/ml einsetzen. Durch Ersatz des ¹⁴C-markierten Acetats durch markiertes Mevalonat konnte bewiesen werden, daß der Angriffspunkt vor der Bildung von Mevalonat lag; Hemmwirkungen, die zu einer Verschiebung des Musters der Vorläufersterine des Cholesterin führen könnten, wurden nicht beobachtet. Die Aktivität der HMG-CoA-Reduktase, des Schlüsselenzyms der Cholesterinsynthese, wird somit indirekt über eine Verstärkung der inaktivierenden oder eine Hemmung der aktivierenden Mechanismen des Enzyms gedrosselt.

Diese Ergebnisse wurden an Primärkulturen menschlicher Hepatocyten bestätigt.

Luteolin und seine Derivate bewirken eine Hemmung der Cholesterin-Neusynthese, reduzieren die Blutfettwerte und wirken somit der Entstehung der Atherosklerose in der Blutgefäßintima direkt entgegen.

Es wurde nachgewiesen, daß Cynarin (1,5-Di-Caffeoyl-D-Chinasäure) in Konzentrationen, die in Artischockenblätterextraktzubereitungen maximal erzielbar sind, keinerlei Hemmung der Cholesterinneusynthese bewirkt. Dies entspricht auch der klinischen Datenlage, wo relevante Lipidsenkungen nur durch die Gabe extremer Dosen synthetischen Cynarins, die im Pflanzenextrakt nicht vorkommen können, erzielt wurden. Cynarin ist zudem als genuiner Bestandteil des Extrakts nur in Spuren enthalten; es entsteht im Verlauf der galenischen Aufbereitung durch Umesterung aus 1,3-Dicaffeoylchinasäure, stellt also ein Artefakt dar.

Die Erfindung betrifft somit die Verwendung von Luteolin der Strukturformel 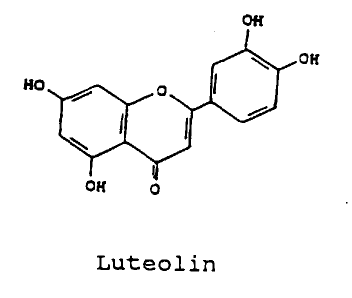 sowie seiner Derivate der allgemeinen Formeln I und II: 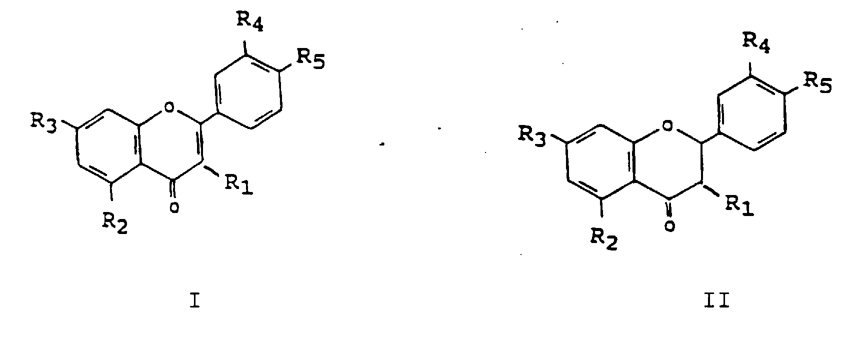 und ihrer pharmazeutisch verträglichen Salze, wobei die Substituenten R₁-R₅ gleich oder verschieden sind und die folgende Bedeutung haben:
-H, -OH, -O-Acyl (z.B. Acetyl), -O-Phenyl, -O-Alkyl (C₁-C₆), -O-Glykosyl (z.B. Glucosyl), -O-Glucuronid,
wobei R₁ keine Hydroxygruppe darstellt, wenn R₂ eine Hydroxygruppe oder ein Wasserstoffatom und R₃-R₅ Hydroxygruppen darstellen zur Herstellung eines Arzneimittels als indirekter Inhibitor der HMG-CoA-Reduktase.

Bevorzugt sind Verbindungen der allgemeinen Formel I und II, in denen R₁-R₅ folgende Bedeutung haben:
a)
   R₁ = H
   R₂ = R₃ = R₄ = R₅ = -OH
b)
   R₁ = H
   R₂ = R₄ = R₅ = -OH
   R₃ = O-Glucosid oder O-Rutinosid
c)
   R₁ = H
   R₂ = R₄ = -OH
   R₃ = O-Rutinosid
   R₅ = O-Glucosid

Aus diesen Verbindungen (b, c) kann Luteolin (a) unter physiologischen Bedingungen (Glykosidspaltung) freigesetzt werden, so daß sie als Prodrugs im Sinne von (a) als Wirkform aufgefaßt werden können.

Die erfindungsgemäß zu verwendenden Verbindungen können in an sich bekannter Weise zu oral oder parenteral verabreichbaren Arzneimittelformen, insbesondere in Form von Kapseln, Tabletten, Granulaten, Tropfen und Ampullenlösungen, verarbeitet werden.

Die erfindungsgemäß verwendeten Verbindungen können auch in Form eines Pflanzenextraktes, beispielsweise eines Artischockenextraktes, verabreicht werden. Vorzugsweise sollte dieser mit dem erfindungsgemäß wirksamen Bestandteil angereichert werden.

Aufgrund seines indirekten Wirkmechanismus kann das Luteolin bzw. seine Derivate in geeigneter Dosierung auch in Form eines Nahrungsmittelergänzungsstoffes verabreicht werden.

Die nachstehenden Beispiele erläutern die Erfindung.

### Beispiel 1

### Angereicherter Extrakt aus frischen Artischockenblättern:

Extraktion der Blätter mit Wasser, Konzentrierung der Extraktbrühe im Vakuum, Fällung von höhermolekularen Verbindungen mit Alkohol, Konzentration des Filtrats im Vakuum, Mischung des Konzentrats mit Polyamidadsorbens, Trocknung, Säulenchromatographie an Polyamid mit Wasser (Vorlauf wird verworfen), Elution phenolischer Verbindungen mit Ethanol-Wasser, Konzentrierung des Eluats im Vakuum bis zum Trockenextrakt. Gehalt an Luteolin und Luteolinglykosiden 5-10%, ber. als Luteolin-7-O-glucosid.

### Beispiel 2

### Angereicherter Extrakt aus Artischockenblätterdroge:

Extraktion der ggf. entfetteten Blattdroge mit Ethanol (40-80%) oder Methanol (50-90%), Konzentrierung der Extraktbrühe im Vakuum, Zusatz von technologischen Hilfsstoffen, Herstelllung einer Trockenextraktzubereitung im Vakuum. Gehalt an Luteolin und Luteolinglykosiden 3-5%, ber. als Luteolin-7-O-glucosid.

### Beispiel 3

### Fertigarzneimittel zur oralen Anwendung:

Tablette oder Filmtablette mit Luteolin, Excipientien (mikrokristalline Cellulose, Lactose, Talkum, Magnesiumstearat, Stärke, hochdisperses Siliciumdioxid, Natriumcarboxymethylstärke, Polyvidon, Macrogol) ad 200 bis 400 mg.

### LITERATURVERZEICHNIS

1. FORTH, W., W. RUMMEL: Pharmakotherapie im Gastrointestinaltrakt. In: Allgemeine und spezielle Pharmakologie und Toxikologie, BI Wissenschaftsverlag, Mannheim, Leipzig, Wien, Zürich (Hsg.: Forth, W.; Henschler, D.; Rummel, W.; Starke, K.) 6. Aufl. Auflage (1992) 466.
2. WEBER (HSG.), E.: Taschenbuch der unerwünschten Arzneiwirkungen. Gustav Fischer, Stuttgart, New York (2. Aufl.) (1988) 465.
3. ASSMANN, G., P. CULLEN: Erkennung und Behandlung von Fettstoffwechselstörungen. Deutsches Ärzteblatt, Suppl. 92 (1995) 2.
4. Cholesterinsynthesehemmer Fluvastatin. arzneitelegramm, Nr. 10 (1994) 96.
5. WINDLER, E., H. GRETEN: Cholesterinsenkung reduziert Herzinfarktrate und Gesamtmortalität. Dt. Ärztebl. 92 (1995) A-1158.
6. SCHWANDT, P.: Hyperlipoproteinämie und Arteriosklerose. Dt. Apotheker Zeitung 133 (1993), 3039.
7. NEATON, J. D. et al.: Serum Cholesterol level and mortality findings for men screened in the Multiple Risk Factor Intervention Trial., Arch Int Med 152 (1992), 1490.
8. RICHTER, W. O.: Die medikamentöse antilipämische Therapie verbessert die Prognose der koronaren Herzkrankheit. Lipidreport 3 (1994) 3.
9. ARNTZ, H.-R.: Kontrollen bei Hyperlipidämie-Behandlung. Z. Allg. Med. 70 (1994), 540.
10. Aktuelle Diskussion: Cholesterin: Wann behandeln? Ärztl. Praxis 15(76) (1988) 2292.
11. Empfehlungen zur Behandlung erhöhter Blutfettwerte arznei-telegramm (12) (1991), 111.
12. Walper, A. et al.: Effizienz einer Diätempfehlung und einer zusätzlichen Phytotherapie mit Allium sativum bei leichter bis mäßiger Hypercholesterinämie. Med. Welt 45 (1994), 327-332.

## Patentansprüche

1. Verwendung von Luteolin und seiner Derivate der Formeln I und II 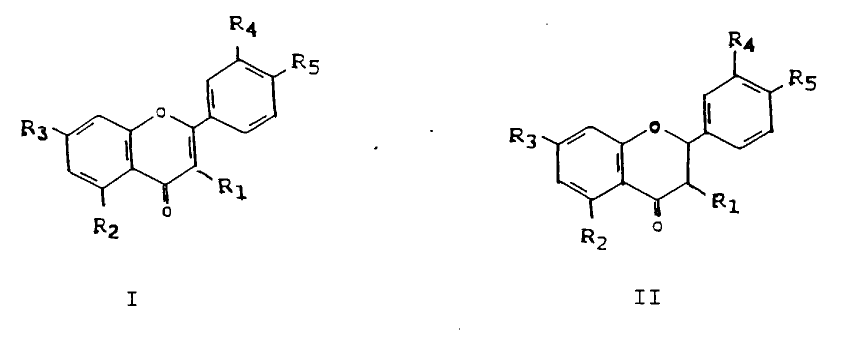 und ihrer pharmazeutisch verträglichen Salze, wobei die Substituenten R₁-R₅ gleich oder verschieden sind und die folgende Bedeutung haben:
-H, -OH, -O-Acyl (z.B. Acetyl), -O-Phenyl, -O-Alkyl (C₁-C₆), -O-Glykosyl (z.B. Glucosyl), -O-Glucuronid,
wobei R₁ keine Hydroxygruppe darstellt, wenn R₂ eine Hydroxygruppe oder ein Wasserstoffatom und R₃-R₅ Hydroxygruppen darstellen zur Herstellung eines Arzneimittels als indirekter Inhibitor der HMG-CoA-Reduktase.

2. Verwendung gemäß Anspruch 1 zur Prophylaxe und Behandlung von leichteren Formen von Dyslipoproteinämien, insbesondere von Hyperlipoproteinämien.

3. Verwendung gemäß Anspruch 1 oder 2 zur Behandlung von Dyslipoproteinämien, bei denen die Anwendung von direkten HMG-CoA-Reduktase-Hemmern nicht in Betracht kommt.

4. Verwendung gemäß Anspruch 1 oder 2 zur Behandlung von Dyslipoproteinämien, bei denen eine Absenkung des Cholesterins unter die Normgrenze (Hypocholesterinämie) vermieden werden muß.

5. Verwendung gemäß Anspruch 1 bis 4 für eine Langzeittherapie.

6. Verwendung gemäß Anspruch 1 bis 5 als Ergänzung oder Substitution einer lipidsenkenden Diät.

7. Verwendung von Luteolin und seinen Derivaten der Formeln I und II, wie sie in Anspruch 1 definiert sind, als Nahrungsmittelergänzungsstoff.
